# EUROPEAN PATENT SPECIFICATION

(11) **EP 1 559 367 B1**
(45) Date of publication and mention of the grant of the patent: **20.08.2008**
(21) Application number: 05001689.8
(22) Date of filing: 27.01.2005
(51) Int. Cl.: A61B 5/0404

(54) **Biological information measurement device connection unit**
Verbindungsmodul für ein Gerät zur Messung biologischer Informationen
Unité de raccordement de dispositif de mesure de l'information biologique

(30) Priority: 30.01.2004 JP 2004022800
(43) Date of publication of application: 03.08.2005
(73) Proprietor: Omron Healthcare Co., Ltd., Kyoto 615-0084 (JP)
(72) Inventor: Umeda, Masahiro Omron Healthcare Co., Ltd., Kyoto-shi Kyoto 615-0084 (JP); Yamamoto, Norihito Omron Healthcare Co., Ltd., Kyoto-shi Kyoto 615-0084 (JP); Ishida, Junichi Omron Healthcare Co., Ltd., Kyoto-shi Kyoto 615-0084 (JP); Tanabe, Kazuhisa Omron Healthcare Co., Ltd., Kyoto-shi Kyoto 615-0084 (JP)
(74) Representative: Kilian, Helmut

(56) References cited:
- EP-A- 1 129 662
- DE-U1- 8 911 113
- DE-U1- 20 306 009

## Description

### BACKGROUND OF THE INVENTION

### Field of the Invention

The present invention relates to a biological information measurement device connection unit used for connecting a biological information measurement device to an external terminal unit, and more particularly to a cradle for a biological information measurement device establishing electrical connection between the biological information measurement device and the external terminal unit while holding the biological information measurement device.

### Description of the Background Art

An electrocardiograph and a body fat meter represent examples of widely known biological information measurement devices having a measurement electrode to be brought into contact with a living body and obtaining biological information by processing a biological electric signal detected by the measurement electrode. As to the biological information measurement device, conventionally, connection to an external terminal unit has strongly been demanded.

In the electrocardiograph, demand for analysis and storage of obtained electrocardiographic data, printout of the data on paper medium, and transmission of the electrocardiographic data to a remote location has been very strong, and it has also been demanded to provide an interface in a device main body for the purpose of establishing connection to a personal computer (hereinafter, abbreviated as "PC"), a printer, or communication means. Such demands are great particularly in a Holter electrocardiograph and a portable electrocardiograph that have widely been used in recent days. Specifically, in order to present the electrocardiographic data obtained at home to a specialist, connection to the external terminal unit has strongly been demanded.

In order to realize connection to the external terminal unit in the electrocardiograph, some kind of interface should be provided in the device main body of the electrocardiograph for communicating a signal between the device main body of the electrocardiograph and the external terminal unit. As the interface, a variety of interfaces such as infrared communication, fiber optics communication, magnetic coupling, inductive coupling, and the like may be adopted (see Japanese Patent Laying-Open Nos. 9-224917, 63-206225, and 5-7560, for example). When such an interface is adopted, however, not only a device structure is complicated, but also cost and performance are not satisfactory. Here, as a connection method to realize ensured communication of a signal with low cost, wired connection using a serial bus such as a USB (Universal Serial Bus) and an RS-232C may be employed.

In wired connection using the USB, for example, an output terminal is provided in the device main body of the electrocardiograph and an input terminal is provided in a main body of the external terminal unit. Then, the output terminal and the input terminal are connected to each other via a USB connection cable, so that connection between the device main body of the electrocardiograph and the external terminal unit is established. In this manner, communication of the signal between the device main body of the electrocardiograph and the external terminal unit is reliably realized with low cost.

Meanwhile, when wired connection using a connection cable is adopted, a power supply voltage input to the external terminal unit may be introduced as a surge into a processing circuit provided inside the device main body of the electrocardiograph through the connection cable. If the power supply voltage is introduced while a subject touches the measurement electrode, the subject may receive electric shock through the measurement electrode. Therefore, considering safety of the subject, some measure to prevent electric shock should be provided in order to avoid such an accident of electric shock.

A commonly known measure to prevent electric shock employs a photocoupler (see Japanese Utility Model Laying-Open No. 5-9507, and Japanese Patent Laying-Open Nos. 61-232832 and 63-272324, for example). The photocoupler optically couples two electrically isolated electric circuits with each other, and the photocoupler is normally implemented by a combination of a light-emitting diode and a phototransistor. When the photocoupler is employed, an internal circuit in the external terminal unit can electrically be isolated from the measurement electrode of the electrocardiograph. Therefore, occurrence of an electric shock accident can be prevented.

In order to provide the electric shock prevention measure in the electrocardiograph by using the photocoupler, however, a plurality of photocouplers (two to four) should be mounted on the device main body of the electrocardiograph. The photocoupler is a relatively expensive element, and mounting of a plurality of photocouplers results in increase in manufacturing cost. In addition, larger size of the device is unavoidable.

Meanwhile, the electric shock prevention measure can be provided in the electrocardiograph having the measurement electrode drawn out of the device main body via the connection cable in the following manner. Specifically, such an electrocardiograph may be structured such that simultaneous connection to the device main body of the electrocardiograph, of the connection cable for establishing connection between the device main body of the electrocardiograph and the measurement electrode and the connection cable for establishing connection between the device main body of the electrocardiograph and the external terminal unit is not allowed (see Japanese Utility Model Laying-Open Nos. 5-9508 and 5-9509, and Japanese Patent Laying-Open No. 6-197875, for example). According to such a structure, while the device main body of the electrocardiograph is connected to the external terminal unit, the processing circuit provided inside the device main body of the electrocardiograph is electrically isolated from the measurement electrode in an ensured manner. Therefore, the accident of electric shock as described above can reliably be avoided.

Though such an electric shock prevention measure as above is extremely effective in the electrocardiograph having the measurement electrode drawn out of the device main body through the connection cable, the electrocardiograph having the measurement electrode provided on an outer surface of the device main body (a type mainly found in portable electrocardiographs) cannot adopt such an electric shock prevention measure, and therefore, another measure should be provided. Document DE-U-8 911 113 discloses an apparatus according to the preamble of claim 1.

### SUMMARY OF THE INVENTION

An object of the present invention is to provide a biological information measurement device connection unit preventing occurrence of an accident of electric shock possible when the biological information measurement device having a measurement electrode provided on an outer surface of a device main body is connected to an external terminal unit.

A biological information measurement device connection unit according to the present invention connects a biological information measurement device to an external terminal unit. The biological information measurement device connection unit includes: a holding portion receiving and holding a device main body of the biological information measurement device; a connection terminal electrically connected to the external terminal unit and connected to an output terminal provided in the device main body of the biological information measurement device while the device main body of the biological information measurement device is attached to the holding portion; and a shield portion covering a measurement electrode provided on an outer surface of the device main body of the biological information measurement device while the device main body of the biological information measurement device is attached to the holding portion.

According to the present invention, the biological information measurement device connection unit reliably preventing occurrence of an accident of electric shock possible when the biological information measurement device having the measurement electrode on the outer surface of the device main body is connected to the external terminal unit can be provided.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic perspective view showing a configuration of a portable electrocardiograph employed in an embodiment of the present invention.
Fig. 2 is a front view of the portable electrocardiograph shown in Fig. 1.
Fig. 3 is a top view of the portable electrocardiograph shown in Fig. 1 when the cover is closed.
Fig. 4 is a bottom view of the portable electrocardiograph shown in Fig. 1.
Fig. 5 is a right side view of the portable electrocardiograph shown in Fig. 1.
Fig. 6 is a left side view of the portable electrocardiograph shown in Fig. 1.
Fig. 7 is a perspective view of a measurement posture to be taken by a subject at the time of measuring an electrocardiographic waveform using the portable electrocardiograph employed in the embodiment of the present invention.
Fig. 8 illustrates a measurement posture to be taken by the subject at the time of measuring an electrocardiographic waveform using the portable electrocardiograph in the embodiment of the present invention, viewed from the above.
Fig. 9 illustrates a state where the portable electrocardiograph employed in the embodiment of the present invention is held with a right hand.
Fig. 10 is a schematic perspective view of a cradle in the embodiment of the present invention.
Fig. 11 is a front view of the cradle in the embodiment of the present invention.
Fig. 12 is a top view of the cradle in the embodiment of the present invention.
Fig. 13 is a right side view of the cradle in the embodiment of the present invention.
Fig. 14 is a schematic perspective view showing a manner of attaching the portable electrocardiograph to the cradle in the embodiment of the present invention.
Fig. 15 is a front view of a state where the portable electrocardiograph is attached to the cradle in the embodiment of the present invention.
Fig. 16 is a right side view of the state where the portable electrocardiograph is attached to the cradle in the embodiment of the present invention.
Fig. 17 is a left side view of the state where the portable electrocardiograph is attached to the cradle in the embodiment of the present invention:
Fig. 18 is a block diagram showing a state where the portable electrocardiograph employed in the embodiment of the present invention is connected to a PC.

### DESCRIPTION OF THE PREFERRED EMBODIMENTS

The inventors have conceived that a cradle is to be adopted as a biological information measurement device connection unit for connecting the biological information measurement device to the external terminal unit, so that convenience in a connection operation is drastically improved and the biological information measurement device is held in a stable manner while it is connected to the external terminal unit. Here, the cradle refers to a frame or a base for supporting or binding a portable terminal and the like. That is, the cradle refers to a portable terminal connection unit that has quickly spread with recent widespread use of the portable terminals represented by a digital camera or a portable phone. Normally, the cradle has a structure for establishing electrical connection between the portable terminal and the external terminal unit as well as for carrying the portable terminal on a desk or the like in a stable manner. The cradle is sometimes called a desktop holder.

On the other hand, unless some measure is taken when such a cradle is adopted as the biological information measurement device connection unit, a surge from a power supply circuit in the external terminal unit may be applied to a processing circuit provided inside the biological information measurement device. If a user touches the device main body of the biological information measurement device for operating the same while the surge is applied to the processing circuit of the biological information measurement device, the user may receive electric shock through the measurement electrode. The inventors have conceived that this problem is solved by providing an electric shock prevention structure in the cradle, and completed the present invention.

In the following, one embodiment of the present invention will be described in detail with reference to the drawings. In the embodiment as set forth below, a portable electrocardiograph that can be carried and can easily measure and store an electrocardiographic waveform will be described by way of example of a biological information measurement device.

Initially, an appearance of the portable electrocardiograph employed in the present embodiment will be described.

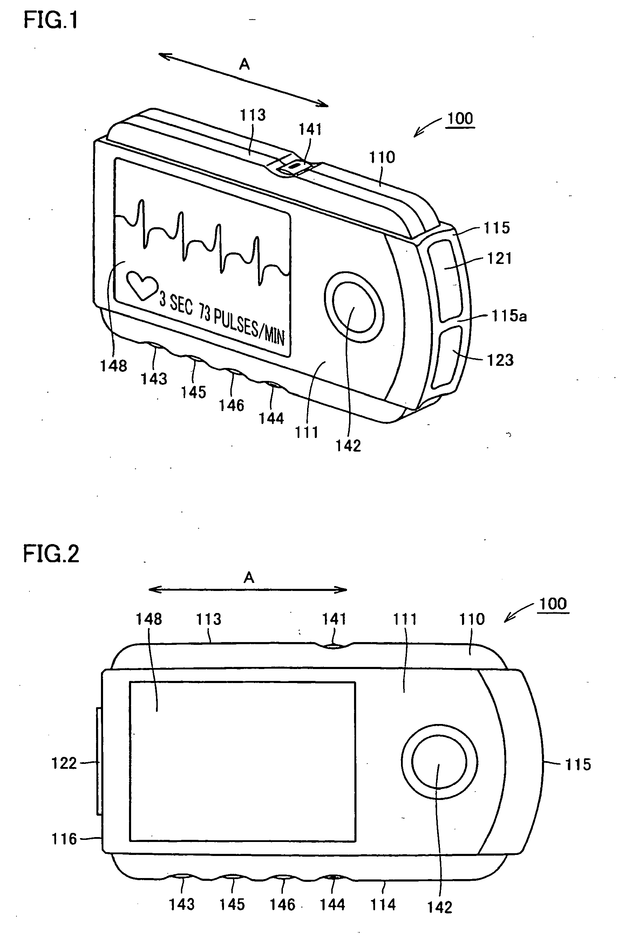

As shown in Figs. 1 to 6, in order to realize excellent usability, a portable electrocardiograph 100 employed in the present embodiment has such a light weight and a small size that it can be held by one hand. Portable electrocardiograph 100 includes a device main body 110 having a flat and elongated, substantially rectangular parallelepiped shape. On its outer surfaces (a front face 111, a rear face 112, a top face 113, a bottom face 114, a right side face 115, and a left side face 116), a display unit, an operation unit, a measurement electrode, and the like are disposed.

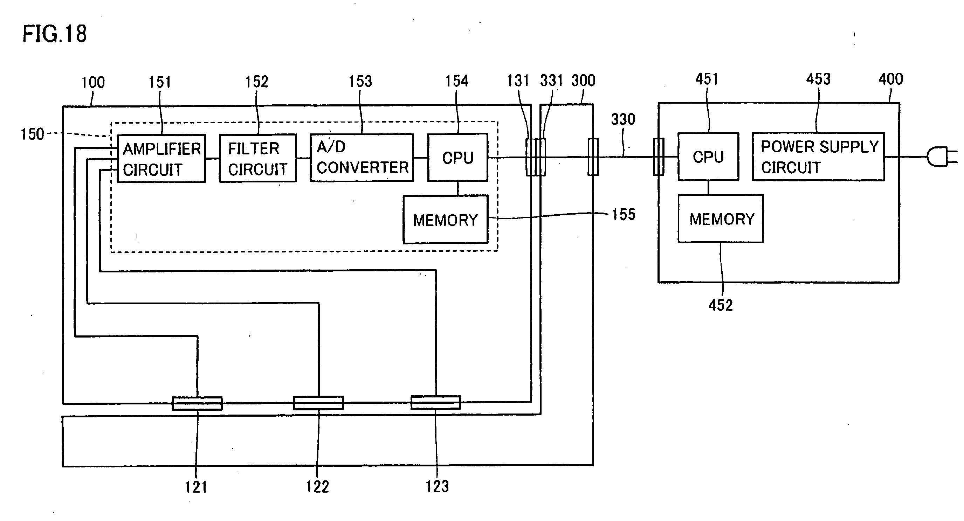

Device main body 110 contains a processing circuit 150 for processing a biological electric signal detected by the measurement electrode (see Fig. 18). Processing circuit 150 includes, for example, an amplifier circuit 151 amplifying the biological electric signal detected by the measurement electrode, a filter circuit 152 removing a noise component from the biological electric signal detected by the measurement electrode, an A/D converter 153 converting an analog signal to a digital signal, a CPU (central processing unit) 154 performing a variety of operations, a memory 155 temporarily storing electrocardiographic information, and the like.

As shown in Figs. 1 and 2, a measurement button 142 serving as an operation button for starting measurement is provided in a portion close to one end in the longitudinal direction (the direction shown with an arrow A in the drawing) of front face 111 of device main body 110. In a portion close to the other end of front face 111 of device main body 110, a display unit 148 is provided. Display unit 148 is implemented, for example, by a liquid crystal display and serves to display a result of measurement or the like. The measurement result is displayed, for example, as electrocardiographic waveforms or numerical data as shown in Fig. 1.

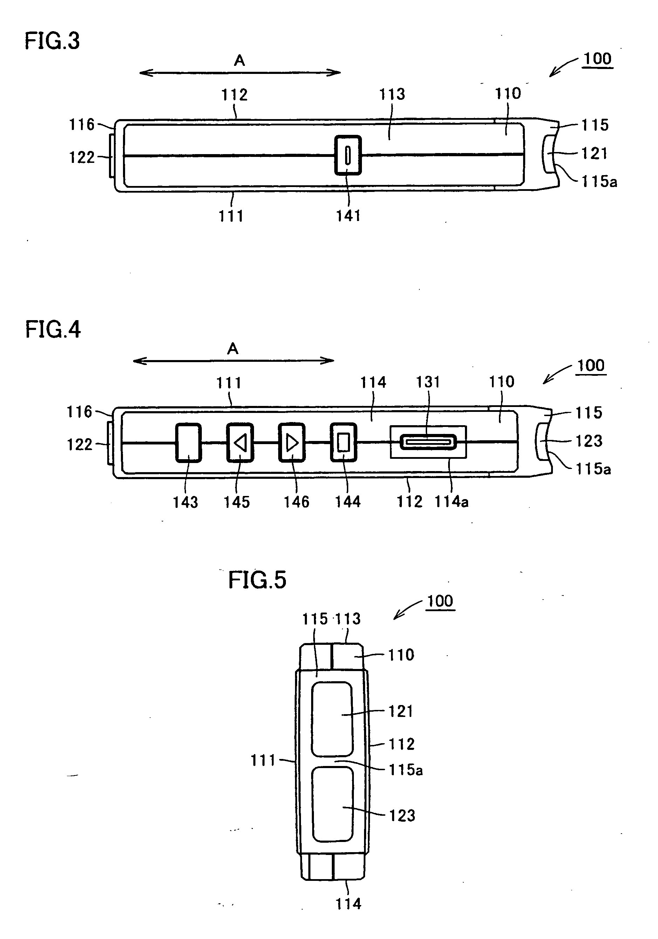

As shown in Figs. 1 and 3, a power button 141 is disposed in a prescribed position in top surface 113 of device main body 110. Power button 141 serves as an operation button for turning ON/OFF portable electrocardiograph 100.

As shown in Figs. 1 and 4, various operation buttons are disposed in prescribed positions on bottom face 114 of device main body 110. In shown portable electrocardiograph 100, a setting button 143, a display button 144, a left scroll button 145, and a right scroll button 146 are disposed. Setting button 143 is used for making a variety of settings for portable electrocardiograph 100, while display button 144 is used for displaying a measurement result on display unit 148. Left scroll button 145 and right scroll button 146 are used for scrolled display of a graph showing a measurement result or guide information displayed on display unit 148.

In addition, as shown in Fig. 4, a concave portion 114a is provided in a prescribed position of bottom face 114 of device main body 110, and an output terminal 131 for establishing connection to the external terminal unit is provided on a bottom surface of concave portion 114a. Output terminal 131 is used for supplying the electrocardiographic information temporarily stored in a memory provided inside device main body 110 to the external terminal unit, and connected to a connection terminal 331 of a cradle 300 which will be described later. Portable electrocardiograph 100 in the present embodiment is characterized by being connected to the external terminal unit via cradle 300. In order to prevent direct connection of portable electrocardiograph 100 to the external terminal unit without cradle 300 being interposed, a terminal having a shape different from a shape of a general-purpose connector such as a USB or an RS-232C is employed as output terminal 131 provided in device main body 110.

As shown in Figs. 1 and 5, on right side face 115 located at one end in the longitudinal direction of device main body 110, a negative electrode 121 representing one electrode out of a pair of measurement electrodes as well as an indifferent electrode 123 for deriving a potential serving as a reference in potential variation in the body are disposed. Right side face 115 has a smoothly curved shape, such that a forefinger of the right hand of the subject is fitted thereto when the subject takes a measurement posture which will be described later. In addition, a concave portion 115a extending in an up-down direction is formed in right side face 115. Concave portion 115a is in a shape to receive the forefinger of the right hand of the subject.

Negative electrode 121 and indifferent electrode 123 described above are formed with a conductive member, and electrically connected to processing circuit 150 provided inside device main body 110. In addition, negative electrode 121 and indifferent electrode 123 are disposed in concave portion 115a provided in right side face 115 such that their surfaces are exposed on the outer surface of device main body 110. Negative electrode 121 is located closer to top face 113 on right side face 115, while indifferent electrode 123 is located closer to bottom face 114 on right side face 115.

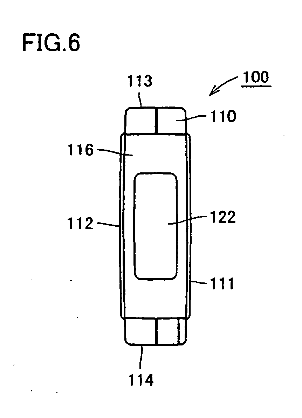

As shown in Fig. 6, on left side face 116 located at the other end in the longitudinal direction of device main body 110, a positive electrode 122 representing the other electrode out of a pair of measurement electrodes is disposed. Positive electrode 122 is formed with a conductive member, and electrically connected to processing circuit 150 provided inside device main body 110.

Next, a measurement posture to be taken by the subject in measuring an electrocardiographic waveform using portable electrocardiograph 100 having the above-described structure will be described.

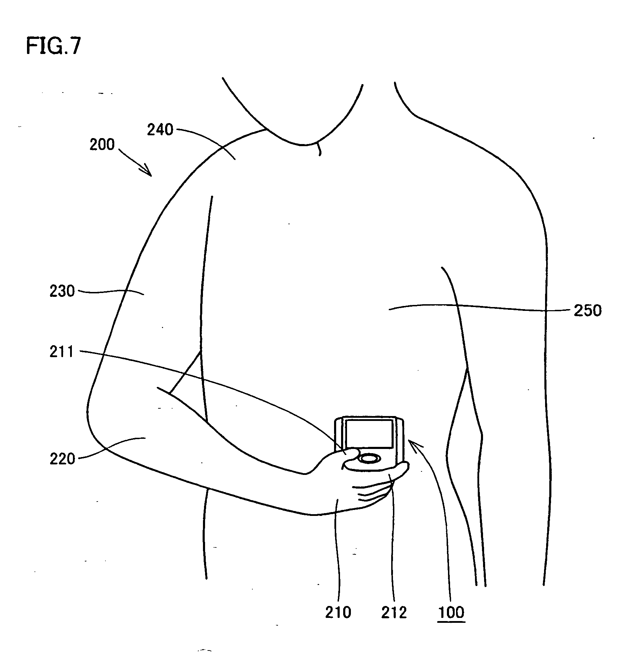

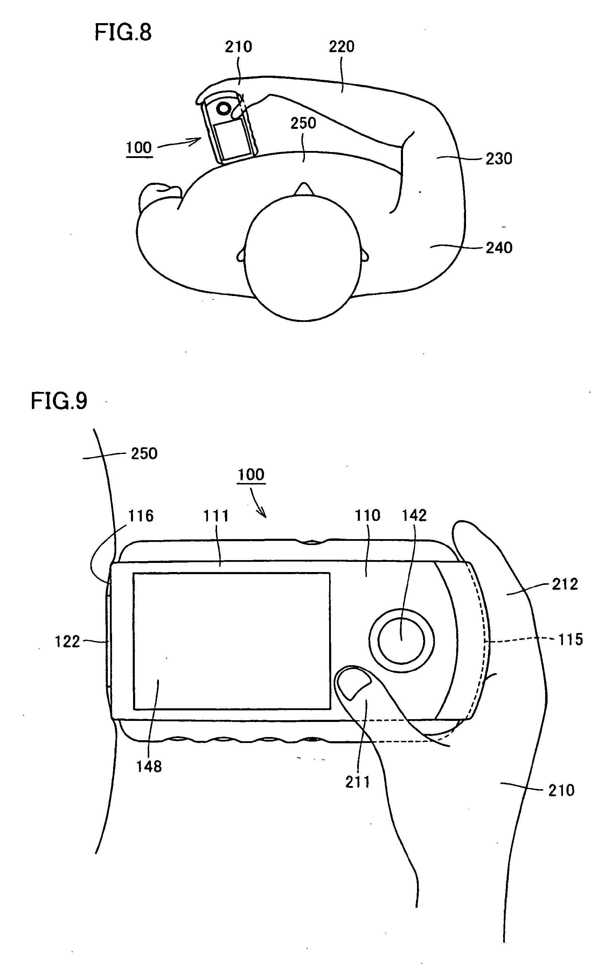

As shown in Figs. 7 and 8, during measurement, a subject 200 holds a portion closer to one end of device main body 110 of portable electrocardiograph 100 with his/her right hand 210, and brings positive electrode 122 provided on left side face 116 ' located at the other end of device main body 110 into contact with the skin on a fifth intercostal anterior axillary line located in a lower left portion of chest 250. Then, the subject presses measurement button 142 provided on front face 111 of device main body 110 with his/her thumb 211 of right hand 210, and maintains this state for several tens of seconds until measurement of the electrocardiographic waveform is completed.

A state that portable electrocardiograph 100 is held with right hand 210 will now be described.

As shown in Fig. 9, in this measurement posture, subject 200 holds with right hand 210 a portion closer to one end in the longitudinal direction of device main body 110 such that front face 111 of device main body 110 of portable electrocardiograph 100 faces upward. Here, right side face 115 of device main body 110 is covered with forefinger 212 of right hand 210, thumb 211 of right hand 210 is placed on front face 111 of device main body 110, and the middle finger of right hand 210 is placed on the rear face of device main body 110. That is, device main body 110 is held such that it is caught by three fingers. In this state, forefinger 212 of right hand 210 is lightly bent such that the forefinger extends along curved right side face 115 and is inserted in concave portion 115a provided in right side face 115. Forefinger 212 of right hand 210 is thus brought into contact with negative electrode 121 and indifferent electrode 123 provided in concave portion 115a.

When such a measurement posture is taken, negative electrode 121 and indifferent electrode 123 located on right side face 115 of device main body 110 of portable electrocardiograph 100 come in contact with forefinger 212 of right hand 210 of subject 200, and positive electrode 122 located on left side face 116 of device main body 110 comes in contact with chest 250 of subject 200. In this manner, a measurement circuit is implemented in the body of the subject by right hand 210 being in contact with negative electrode 121, a forearm 220 without contacting chest 250, a brachium 230 and a right shoulder 240 without contacting chest 250, and chest 250 to which positive electrode 122 is attached, in this order.

As described above, an action potential produced by an activity of cardiac muscle is detected as a potential difference between negative electrode 121 and positive electrode 122 serving as the measurement electrodes, and processed by processing circuit 150 provided inside portable electrocardiograph 100. Electrocardiographic information including the electrocardiographic waveform can thus be obtained. The electrocardiographic information obtained in such a manner is temporarily stored in memory 155 provided inside portable electrocardiograph 100 and output to the external terminal unit when necessary.

A structure of a cradle in the present embodiment will now be described in detail.

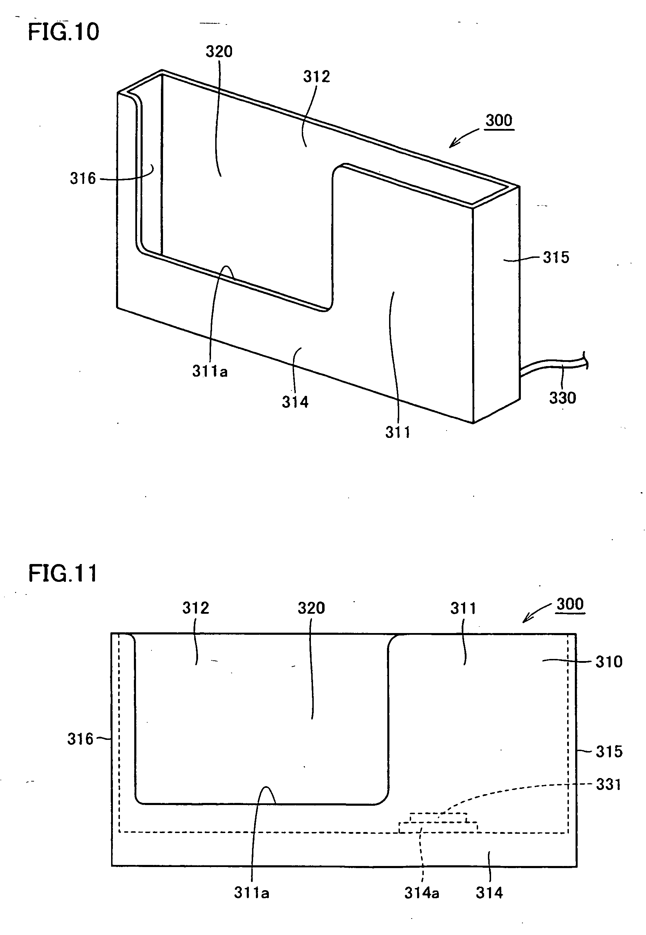

As shown in Figs. 10 to 13, cradle 300 has a box-shaped frame body 310 having an opening in one face. Frame body 310 includes a bottom portion 314 placed on a carrier surface such as a desk, as well as a front wall, 311, a rear wall 312, a right sidewall 315, and a left sidewall 315 rising from four sides of bottom portion 314. Frame body 310 includes a holding portion 320 for receiving and holding device main body 110 of portable electrocardiograph 100. The opening described above is provided for attaching portable electrocardiograph 100 to cradle 300, and located in an upper portion of frame body 310. Frame body 310 is formed with a molded product made of an insulating resin material, for example.

As shown in Figs. 10 and 11, a notch 311 a is provided in a prescribed position of front wall 311 of frame body 310. Notch 311 a serves as a window exposing display unit 148 provided on the outer surface of portable electrocardiograph 100 while portable electrocardiograph 100 is attached to cradle 300.

As shown in Figs. 11 to 13, a convex portion 314a is provided in a prescribed position of an upper surface of bottom portion 314 of frame body 310. Convex portion 314a fits to concave portion 114a provided in bottom face 114 of device main body 110 of portable electrocardiograph 100 described above. In addition, connection terminal 331 is provided on a top surface of convex portion 314a. Connection terminal 331 is connected to output terminal 131 provided in device main body 110 of portable electrocardiograph 100 while portable electrocardiograph 100 is attached to cradle 300. Here, a terminal having a shape different from a shape of a general-purpose connector such as a USB or an RS-232C is employed as connection terminal 331, as set forth above.

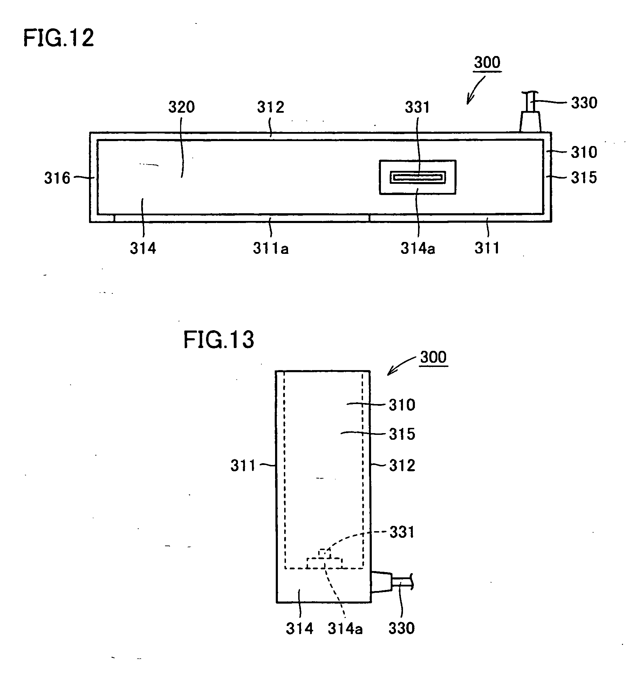

As shown in Figs. 12 and 13, a connection cable 330 is drawn out from a prescribed position in rear wall 312 of frame body 310 to the outside of frame body 310. Connection cable 330 is electrically connected to connection terminal 331 described above via a relay circuit (not shown) formed inside frame body 310, and has the other end connected to an input terminal of the external terminal unit.

Cradle 300 in the present embodiment establishes electrical connection with the external terminal unit by accommodating device main body 110 of portable electrocardiograph 100 described above in holding portion 320. In other words, by attaching portable electrocardiograph 100 to cradle 300, the electrocardiographic information temporarily stored in memory 155 provided inside device main body 110 of portable electrocardiograph 100 can be output to the external terminal unit.

In the following, a procedure for attaching portable electrocardiograph 100 to cradle 300 in the present embodiment as well as a structure of cradle 300 and portable electrocardiograph 100 after attachment is completed will be described in detail.

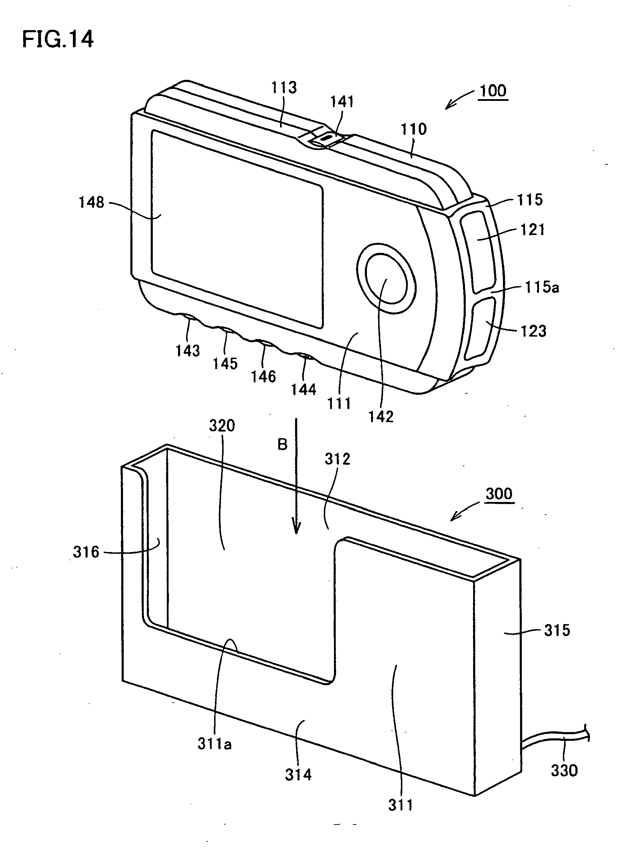

As shown in Fig. 14, when portable electrocardiograph 100 is attached to cradle 300, cradle 300 is placed on the carrier surface such as a desk in advance, and device main body 110 of portable electrocardiograph 100 is inserted into holding portion 320 through the opening formed in the upper portion of placed cradle 300 (in a direction of an arrow B in the drawing). Here, device main body 110 of portable electrocardiograph 100 is guided by front wall, 311, rear wall 312, right sidewall 315, and left sidewall 316 of frame body 310 of cradle 300, and positioned and inserted in holding portion 320. Then, bottom face 114 of portable electrocardiograph 100 abuts on the upper surface of bottom portion 314 of frame body 310, and attachment of portable electrocardiograph 100 to cradle 300 is thus completed.

When portable electrocardiograph 100 is attached to cradle 300 through the attachment procedure described above, portable electrocardiograph 100 is held by cradle 300 in a stable manner. Therefore, falling of portable electrocardiograph 100 by inadvertently touching portable electrocardiograph 100 during an operation or falling of portable electrocardiograph 100 from the carrier surface such as a desk down to a floor or the like is prevented. Consequently, breakage of portable electrocardiograph 100 is prevented.

As described above, convex portion 314a provided on the upper surface of bottom portion 314 of cradle 300 is positioned and formed in a position corresponding to concave portion 114a provided on bottom face 114 of portable electrocardiograph 100 while portable electrocardiograph 100 is attached to cradle 300. Therefore, the attachment operation as above allows smooth fitting of convex portion 314a and concave portion 114a. As a result, connection between output terminal 131 provided in bottom face 114 of portable electrocardiograph 100 and connection terminal 331 provided on the upper surface of bottom portion 314 of cradle 300 when the portable electrocardiograph is attached to the cradle can be ensured.

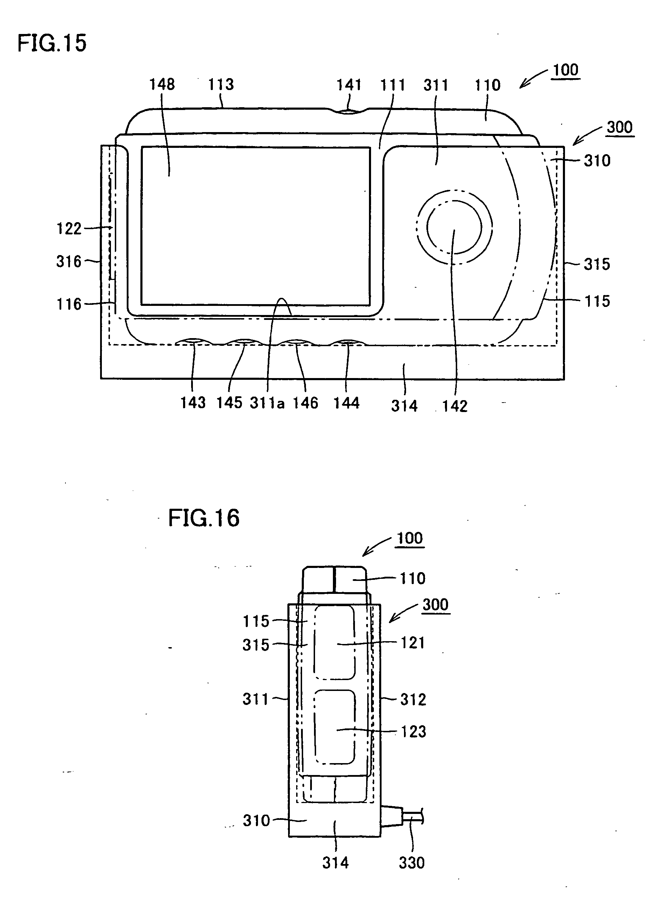

In addition, as shown in Fig. 15, while portable electrocardiograph 100 is attached to cradle 300, display unit 148 provided in front face 111 of device main body 110 of portable electrocardiograph 100 is exposed through notch 311a formed in front wall 311 of frame body 310 of cradle 300. Therefore, display unit 148 can visually be recognized when the portable electrocardiograph is connected to the external terminal unit.

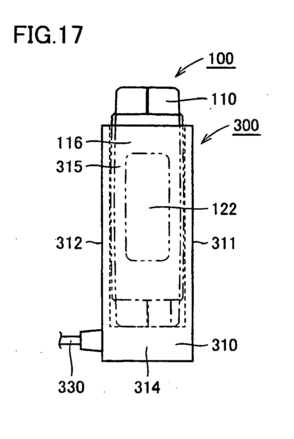

As shown in Figs. 16 and 17, while portable electrocardiograph 100 is attached to cradle 300, negative electrode 121 and indifferent electrode 123 provided on right side face 115 of portable electrocardiograph 100 are covered with right sidewall 315 of cradle 300, and positive electrode 122 provided on left side face 116 of portable electrocardiograph 100 is covered with left sidewall 316 of cradle 300. That is, right sidewall 315 and left sidewall 316 of cradle 300 serve as the shield portion covering negative electrode 121, positive electrode 122 and indifferent electrode 123 provided on the outer surface of portable electrocardiograph 100, so that the user cannot directly touch these electrodes when the portable electrocardiograph is attached to the cradle.

A system configuration when portable electrocardiograph 100 employed in the present embodiment is connected to the external terminal unit and the electrocardiographic information stored in memory 155 is output to the external terminal unit will now be described. It is noted that the system configuration as set forth below employs a PC 400 as the external terminal unit connected to device main body 110 of portable electrocardiograph 100 by way of example.

As shown in Fig. 18, when portable electrocardiograph 100 is connected to PC 400 representing the external terminal unit, device main body 110 of portable electrocardiograph 100 is attached to holding portion 320 of cradle 300. Thus, device main body 110 of portable electrocardiograph 100 is connected to PC 400 via the relay circuit provided in cradle 300 and connection cable 330, and processing circuit 150 provided inside device main body 110 of portable electrocardiograph 100 is electrically connected to an internal circuit of PC 400 (a CPU 451, a memory 452, and the like). In this manner, communication of a signal between processing circuit 150 provided inside device main body 110 of portable electrocardiograph 100 and the internal circuit provided inside PC 400 is allowed, so that the electrocardiographic information stored in memory 155 of portable electrocardiograph 100 can be transferred to memory 452 in PC 400.

As described above, cradle 300 in the present embodiment includes holding portion 320 receiving and holding device main body 110 of portable electrocardiograph 100, connection terminal 331 electrically connected to PC 400 representing the external terminal unit and connected to output terminal 131 provided in device main body 110 of portable electrocardiograph 100 while device main body 110 of portable electrocardiograph 100 is attached to holding portion 320, and the shield portion (corresponding to right sidewall 315 and left sidewall 316) covering negative electrode 121, positive electrode 122 and indifferent electrode 123 provided on the outer surface of device main body 110 of portable electrocardiograph 100 while device main body 110 of portable electrocardiograph 100 is attached to holding portion 320.

Therefore, when device main body 110 of portable electrocardiograph 100 is attached to cradle 300 in order to connect portable electrocardiograph 100 to PC 400, the shield portion covers the electrodes provided on the outer surface of portable electrocardiograph 100 without fail. Consequently, even if a power supply voltage from a power supply circuit 453 in PC 400 should be introduced as surge into processing circuit 150 of portable electrocardiograph 100 through the internal circuit in PC 400 and connection cable 330, reception of electric shock by the user can be prevented. Therefore, occurrence of an accident of electric shock can reliably be prevented.

In addition, as it is not necessary to provide a structure for preventing electric shock in particular in portable electrocardiograph 100, there is no concern about poorer portability due to larger size of portable electrocardiograph 100. Furthermore, as the electric shock prevention measure is implemented in cradle 300 solely by modifying a shape of frame body 310, considerable increase in manufacturing cost is not caused.

In the present embodiment described above, a structure in which the signal is output solely from the portable electrocardiograph to the external terminal unit has been described. It is naturally possible, however, to attain a structure allowing input and output of the signal in a bidirectional manner. In addition, the external terminal unit to be connected is not limited to the PC, and a variety of terminal units such as a printer and a communication device are applicable.

Furthermore, in the present embodiment above, the portable electrocardiograph having the measurement electrode and the indifferent electrode provided on the outer surface of the device main body has been described by way of example. An application of the present invention, however, is not limited to the electrocardiograph. The present invention is applicable to any biological information measurement device having a measurement electrode to be brought into contact with a living body provided on the outer surface of the device main body and obtaining biological information by processing a biological electric signal detected by the measurement electrode. Examples of the biological information measurement device include a measurement device to measure a myoelectric potential, brain wave, living body impedance (impedance of internal tissue or skin), or the like.

Moreover, in the present embodiment above, an example in which a cradle attaining a function as a carrier is adopted as a biological information measurement device connection unit has been described. Application of the present invention, however, is not limited to the cradle in particular. For example, the present invention may be applicable to a case-type connection unit attached so as to simply cover the biological information measurement device without assuming placement on the carrier surface such as a desk. Alternatively, the present invention may be applicable to an external terminal unit including, as a part of its housing, a holding portion which receives the device main body of the biological information measurement device as it is.

Although the present invention has been described and illustrated in detail, it is clearly understood that the same is by way of illustration and example only and is not to be taken by way of limitation, the scope of the present invention being limited only by the terms of the appended claims.

## Claims

1. A biological information measurement device connection unit for connecting a biological information measurement device to an external terminal unit, comprising:
a holding portion (320) receiving and holding a device main body of the biological information measurement device;
a connection terminal (331) electrically connected to the external terminal unit and connected to an output terminal provided in the device main body of the biological information measurement device while the device main body of the biological information measurement device is attached to said holding portion (320); **characterized by**
a shield portion (315, 316) covering a measurement electrode provided on an outer surface of the device main body of the biological information measurement device while the device main body of the biological information measurement device is attached to said holding portion (320).

## Patentansprüche

1. Anschlusseinheit für eine Vorrichtung zur Messung biologischer Information für ein Verbinden einer Vorrichtung zur Messung biologischer Information mit einer externen Endgeräteinheit, aufweisend:
einen Halteabschnitt (320), welcher einen Vorrichtungsgrundkörper der Vorrichtung zur Messung biologischer Information aufnimmt und hält;
einen Verbindungsanschluss (331), der mit der externen Endgeräteinheit elektrisch verbunden ist und der mit einem in dem Vorrichtungsgrundkörper der Vorrichtung zur Messung biologischer Information vorgesehenen Ausgangsanschluss verbunden ist, während der Vorrichtungsgrundkörper der Vorrichtung zur Messung biologischer Information an dem Halteabschnitt (320) angebracht ist; **gekennzeichnet durch**
einen Abschirmungsabschnitt (315, 316), der eine an einer Au-βenfläche des Vorrichtungsgrundkörpers der Vorrichtung zur Messung biologischer Information vorgesehene Elektrode abdeckt, während der Vorrichtungsgrundkörper der Vorrichtung zur Messung biologischer Information an dem Halteabschnitt (320) angebracht ist.

## Revendications

1. Unité de connexion d'un dispositif de mesure d'informations biologiques pour la connexion d'un dispositif de mesure des informations biologiques à une unité terminale externe, comprenant :
◆ une portion de support (320) recevant et maintenant un corps principal de dispositif du dispositif de mesure d'informations biologiques ;
◆ un terminal de connexion (331) raccordé électriquement à l'unité terminale externe et connecté à un terminal de sortie situé dans le corps principal de dispositif du dispositif de mesure d'informations biologiques, tandis que le corps principal de dispositif du dispositif de mesure d'informations biologiques est fixé à ladite portion de support (320), **caractérisée par**
◆ une portion de protection (315, 316) couvrant une électrode de mesure fournie sur une surface extérieure du corps principal de dispositif du dispositif de mesure d'informations biologiques, tandis que le corps principal de dispositif du dispositif de mesure d'informations biologiques est fixé à ladite portion de support (320).
